(19) **Europäisches Patentamt**
**European Patent Office**
**Office européen des brevets**

(11) **EP 4 672 260 A1**

(12) **EUROPEAN PATENT APPLICATION**

(43) Date of publication:
**31.12.2025 Bulletin 2026/01**

(21) Application number: **24183908.3**

(22) Date of filing: **24.06.2024**

(51) International Patent Classification (IPC):
**G16H 50/20** (2018.01)     **G16H 50/30** (2018.01)
**G16H 50/70** (2018.01)

(52) Cooperative Patent Classification (CPC):
**G16H 50/30; G16H 50/20; G16H 50/70**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Siemens Healthineers AG
91301 Forchheim (DE)**

(72) Inventors:
• **SCHMUECKING, Ingo
Yardley, 19067 (US)**

• **SHARMA, Puneet
Princeton Junction, 08550 (US)**
• **BHATTACHARYYA, Arijit
91058 Erlangen (DE)**

(74) Representative: **Schwarz, Claudia
Schwarz + Kollegen
Patentanwälte
Heilmannstraße 19
81479 München (DE)**

Remarks:
Amended claims in accordance with Rule 137(2)
EPC.

(54) **AI-BASED CALCULATION OF A CASE COMPLEXITY INDEX**

(57) The present invention relates to an AI-based calculation of a case complexity index, CCI. For training a neural network, NN, the method (100) may comprise receiving (S102) training data, comprising: a medical image (I) of a set of medical images and optionally a related report (R) for the medical image; and a CCI for the medical image (I). The method (100) may further comprise training (S103) the NN for providing a trained NN, which is configured for determining the CCI for a medical image (I) by adjusting weights and biases of the NN such that a loss function is minimized.

FIG 1

**EP 4 672 260 A1**

**Description**

The present invention refers to the field of medical image processing and related tasks and relates to the determination of a case complexity index for use in processing of medical images or related tasks based on the medical images in the field of medical technology. In particular, the present invention relates to a method for training a neural network, NN, to a method for calculating a case complexity index, CCI, for an input dataset, in particular a pair, comprising at least one medical image and a related report by using the trained neural network, NN, to a CCI calculator device, to a computer system and a computer program product.

Imaging service providers, e.g. radiology departments, teleradiology companies, radiology as a service (RaaS) providers, need to optimize efficiency and throughput of image reading and reporting while achieving best possible quality of care (clinical-economical optimization). This requires assignment of cases to the right readers and overall reading worklist prioritization. It also requires precise understanding of the effort (time and difficulty) required to accomplish reading or other image-related tasks.

This represents a complex problem for imaging service providers with high volumes (100k - 1m+ procedures per year), broad procedure mix, and a large pool of radiologists with varying levels of experience and skills.

Imaging exams, such as chest imaging, have a small set of common findings and a large set of uncommon and rare findings known as "long-tail" distribution. There are about 260 imaging findings in chest imaging caused by 2958 different disorders (see e.g., Kahn CE. The Long Tail. Radiology Artificial Intelligence. Posted April 18, 2019, https://pubs.rsna.org/page/ai/blog/2019/4/the_long tail.). An imaging service provider with a pool of general and chest radiologists may want to assign "easier" chest radiographs (e.g. with common findings) to general radiologists and free up chest radiologists to read chest CT exams while also handling "more difficult" chest radiographs (e.g. from the long-tail distribution) to make sure that they are accurately interpreted in case of rare disorders.

Therefore, there is a need in the art to provide an objective basis to control image-based subsequent tasks, like e.g., a reading task.

The imaging IT environment used by radiology providers typically includes a reading worklist. The reading worklist enables physicians to see unread imaging studies including patient information, imaging modality, procedure type, diagnosis/reason for the exam, referring physician, and flags indicating urgent exams. The worklist may also display how long the study has been unread and when it needs to be read as per turnaround time agreements. Throughput and efficiency statistics can be generated including number of cases read, reading times etc.

With workflow orchestration software, it is possible to efficiently implement a wider set of processing rules to determine the assignment of cases to reading physicians. However, these approaches are typically made without analysis of the actual imaging data which need to be read.

In clinical practice, physicians may pick cases from the worklist based on their preferences, e.g. less experienced physicians may choose "easier" cases based on a few simple criteria, such as age, care setting, referrer. Also, it may occur that physicians pick procedures with higher reimbursement over procedures with low reimbursement, which may lead to a non-optimal assignment of the medical images to a medical practitioner and/or to a computing device with appropriate computing resources.

In state of the art, AI tools such as computer-aided triage and notification (CADt) software can be used to analyze specific imaging studies to mark cases with urgent findings. These studies can be prioritized over other studies not marked by the software. However, this does not reduce the overall effort involved in reading the entire worklist.

AI tools may also be used after image reading and reporting is completed to support quality assurance tasks. However, this is a retrospective approach after the radiology service is provided.

None of these approaches discloses a method for providing an objective metric, based on measurement data, relating to the medical images for controlling subsequent image-based processing tasks, like reading tasks, assignment to computing devices etc.

The technical problem of the present invention is to provide means for improving the image-based processing, in particular with respect to time and/or quality.

The problem is solved by means of the subject matter of the independent claims. Further embodiments are represented in the dependent claims.

In the following, the invention will be described with respect to the claimed methods (training and inference) first. Features, advantages or alternative embodiments, mentioned with respect to the methods can be assigned to the other claimed objects (e.g. the computer program or a device, in particular the CCI-calculator device, or system or a computer program product) and vice versa. In other words, the system, apparatus or device (claims) can be improved with features described or claimed in the context of the method and vice versa. In this case, the functional features of the method are embodied by structural units of the apparatus or device or system and vice versa, respectively. The method may refer to a software implementation and the device may refer to a hardware implementation. Generally, in computer science a software implementation and a corresponding hardware implementation (e.g. as an embedded system) are equivalent. Thus, for example, a method step for "receiving input data" may be performed with an input interface (and as known for a person skilled in the art with respective instructions to read data). For the sake of avoiding redundancy, although the device may also be used in the alternative embodiments described with reference to the method, these embodiments are not explicitly described again for the device. In principle, the respective device or apparatus claim is configured to carry out the claimed method.

According to a first aspect the present invention relates to a computer-implemented method for training a neural network, NN, for determining a case complexity index, CCI, comprising the following method steps:

- receiving training data, comprising:

  - a medical image of a set of medical images and optionally a report for the medical image;
  - a CCI for the medical image;

- training the NN for providing a trained NN, which is configured for determining the CCI for a medical image and optionally a related report by adjusting weights and biases of the NN such that a loss function is minimized.

The CCI may be calculated based on a contradicting score. The contradicting score in turn may be calculated on an evaluation of a degree of complementary and/or contradicting and/or inconsistent information between the medical image and the report. A rule, stored in a rule database may be used to apply a function for automatically calculating the contradicting score.

In particular, the higher the degree of complementarity and/or contradiction and/or inconsistency between the medical image and the report, the higher the CCI.

In an embodiment the CCI may be determined by using the formula:

$$CCI = k/cs,$$

wherein cs represents the contradicting score and k represents a constant factor for controlling the degree of proportionality

The CCI may be calculated on the basis of time for reading the medical images (reading time) and/or a difficulty level. The difficulty level may relate to the difficulty for interpreting the medical images and/or may be calculated by means of a difficulty estimator.

The difficulty estimator may be or comprise a machine learning method. The difficulty estimator may be trained on data with ground truth provided by clinical experts based several attributes. The attributes may be selected from the list consisting of:

- Reason for exam,
- Number of prior exams as an indicator of complexity,
- Number of images which need to be reviewed,
- Number of findings: studies no findings, single findings or multiple findings,
- Tasks that need to be performed: e.g. lung nodules measurements with a diameter on axial slices requires less effort than volumetric lung nodule measurement, and
- Quality assurance (QA) data: As part of quality assurance, radiology providers select a sample of studies and have them read by a different physician who indicates agreement with the original report, minor disagreement or major disagreement. The QA result can be used as a parameter to estimate case difficulty.

The CCI is an index for indicating a complexity for a downstream task to be executed on the medical image, in particular a reading task. The reading task may comprise an annotation task.

The CCI may be represented as a digit or figure. The CCI may be normalized in particular as a figure in a value range, e.g. in an interval between 0 and 100. The CCI may serve as metric to assess complexity of image processing. The CCI may e.g., represent an estimated execution time of an annotation task (e.g. a reading time). Alternatively or in addition the CCI may represent a difficulty level of the annotation task (e.g., indicating a required skill or education level of the annotator, also denoted as clinician skill level).

In an optional embodiment it is possible to store the image data differently depending on the calculated CCI. In particular, the complex cases are stored additionally on a special computer and the less complex ones only in the cloud.

In addition or alternatively, subsequent processing of the medical images may be controlled differently based on the calculated CCI. For example, the assignment of the medical images to medical professionals and/or the processing tools to be used for processing the medical images may be controlled differently. In particular, the scenarios may be as follows:

- Cases may be assigned to different clinicians in the reading worklist depending on the CCI;
- Reading worklists across clinicians may be balanced depending on the CCI to make sure that all clinicians manage comparable case difficulty;
- Cases may be processed with different AI tools depending on the CCI, e.g. cases with low difficulty may not need to be processed by a task-specific AI tool and cases with high complexity may get processed by a task-specific AI tool;
- Depending on the CCI, studies may be selected for a pre-annotation service which provides preliminary report and annotations for review by the radiologist who will finalize the report;
- Depending on the CCI, studies may be assigned to different pre-annotation service providers and/or
- Depending on the CCI, some images may be routed to an AI-only read, while other may have a human expert in the loop. In particular, a threshold may be defined for the different routings.

The CCI may serve to calculate a configuration setting and/or a prioritization for a downstream task, such as an annotation task for a set of images.

The training data comprise a medical image.

Optionally the training data may comprise a medical image and a related report in addition to an associated CCI. In this embodiment, the training dataset comprises a pair of "image and related report". The pair is a multi-modal dataset. The pair comprises two different elements, which are acquired from different data sources (e.g. a reporting computer and an image acquisition device or a storage means for images). The pair is a digital dataset that may be stored in a tuple form or as a two-element matrix, comprising at least a medical image and a report related to this image. Typically, the elements of the pair are provided in different formats. The image is provided in an image format for medical images, in particular in a DICOM format and/or the report may be provided in a text format. Radiology reports are typically text based without specific formats. They can be created using free text and/or structured reporting which provides a more controlled structure or vocabulary. They are typically structured having sections, such as:

- Clinical history (reason for the exam)
- Prior comparison: No or yes (exam and date)
- Findings and/or
- Impression.

Alternatively or in addition, the training method and/or the method for calculating the CCI may use a difficulty estimator. The difficulty estimator may be configured to estimate the difficulty or complexity to further process the image. The difficulty estimator may be related to an image and/or to a downstream task and/or may serve to assess the difficulty and/or complexity of subsequent tasks, like reading, annotating, reporting and/or other software-supported tasks to be executed on said image. The difficulty estimator may use information from different sections (in particular in the image, like e.g., meta data, DICOM header data) to estimate the difficulty/complexity. The difficulty estimator may be implemented algorithmically or may be implemented as machine learning algorithm.

The NN which is trained for determining the CCI may have a feedforward architecture, like a perceptron architecture with an input layer, a hidden layer and an output layer. However, it is also possible to use different architectures, such as recurrent neural networks (RNNs) and convolutional neural networks (CNNs), which have different structures and are suited for particular types of (input) data and (e.g., annotation) tasks.

The NN may be a Vision Language Model that can take as input the image and text report simultaneously, and use dedicated image-only and text-only encoders with contrastive learning. Alternatively, the NN can also have joint embeddings of image and text.

The NN may be configured for a regression task, in particular for determination of the CCI.

Typically, each connection between nodes in adjacent layers is associated with a weight, which determines the strength of the connection. During the training process, these weights are adjusted based on the error between the predicted output and the actual output. For this purpose, known techniques may be used like backpropagation and gradient descent.

The loss function (or objective function) is used to measure the difference between the predicted values of a model (CCI for the pair) and the actual values in the training data. For example, a Mean Squared Error (MSE) for quadratic penalization of the model failures, a Mean Absolute Error (MAE) for penalizing errors linearly, which makes it less sensitive to outliers compared to MSE may be used. But also other loss functions, like the Huber Loss, cross-entropy loss/log loss may be applied.

The term image data refers to medical images, in particular provided in a DICOM format. The images may be acquired by different imaging procedures, like CT, x ray, tomosynthesis, MRI, PET, SPECT, ultrasound and others. The images typically represent an anatomical structure of a patient, like e.g., chest, like chest CT and/or chest radiographs. The image may alternatively refer to other anatomical structures, like the heart, liver, intestine etc.

Alternatively or cumulatively the training data may further comprise at least one of the following data elements:

- clinical data for the respective patient, the set of medical images refers to;
- operational data and/or
- guideline data.

Clinical data may refer to data defining the medical task and/or medical setting. Clinical data may comprise a medical question (e.g., exclusion of a pathology, diagnoses of a particular disease etc.), known diagnoses, available documentation for the patient (to whom the image refers to), and/or prior images (e.g. prior studies and/or prior reports), and/or available medical data, like lab data. Lab data refer to data of a medical laboratory analysis, comprising biochemical, image-based, tumor-marker-based, hormonal, genetical, and/or microbiological analysis, of e.g. blood or other body fluids. Alternatively or in addition, clinical data may comprise demographic data, co-morbidities, and/or therapy related data, like already executed therapies (surgical intervention, chemo therapy etc.).

Operational data may refer to data defining the procedure which is to be executed on the medical image data. Operational data may comprise time data, indicating how much time does it take to execute the procedure. In addition or alternatively, operational data may comprise storage data, indicating from which source and/or storage the image data are provided, which type of downstream task is to be executed or expected to be executed on the image data, like e.g. a reporting task, an annotation task etc.

Guideline data may refer to data, provided in medical guidelines, such as guidelines of the WHO (World Health Organization), AHA (American Heart Association) and/or guidelines of the SCCM (Society of Critical Care Medicine). The SCCM produces guidelines for the management of critically ill patients in intensive care units (ICUs). Guideline data may be based on the provisions of the Radiological Society of North America (RSNA). For example, an example for guidelines for diagnostic imaging may , e.g., be the Guidelines for Management of Incidental Pulmonary Nodules Detected on CT Images: From the Fleischner Society 2017. https://pubmed.ncbi.nlm.nih.gov/28240562/.

Alternatively or in addition, the guideline data may be a local/organization-level SOP (standard operating procedure).

Guidelines data may comprise glossary data, like e.g., SNOMED for calibration or harmonization of terminology.

Guidelines data may be actualized according to a predefined update setting. This has the technical advantage that the NN is forced to be trained on actualized guidelines data.

A calibration algorithm may have the functionality to map different terms having the same meaning (like e.g., disease, illness) to a concurring term. The calibration algorithm may use guidelines or guideline data.

Alternatively or in addition, the CCI may be used for configuring or controlling an image annotation task, in particular with respect to estimated reading time, and/or clinician skill level. Alternatively or in addition, the set of medical images may comprise current images and/or prior images of the same or of a related procedure, in particular of the same patient.

In a preferred embodiment, the images in the set of medical images refer to the same patient.

Alternatively, it is possible that the images in the set of medical images refer to other patients with comparable settings. The comparable settings may comprise the type of procedure to be executed on the medical image and/or the type of annotation task to be executed on the image. Alternatively or in addition, comparable settings may also include clinical settings, e.g. outpatient, inpatient, intensive care unit or general hospital vs specialty hospital.

Alternatively or in addition, the method may comprise:

- Using a calibration module, which is configured for calibrating the received training data, in particular with respect to different images and/or reports.

It is to be noted that the calibration module may host different instances of a calibration algorithm which is configured for executing the step of calibrating. One instance may refer to calibrating the training data, as mentioned above and another instance may refer to calibrating input data in inference phase. This could be used as preprocessing for the input data in order to map the terms of the textual input data into terms which were used during training. In general, "calibration" refers to both instances and may relate to a calibration algorithm.

Alternatively or in addition, calibration may relate to calibrate different types of reports, comprising different level of details, different terminology used, different wordings of the authors of the report and/or different structuring, like a structured report versus a free text report.

Calibration may alternatively of in addition relate to a location-based calibration, e.g., a calibration for different medical institutions at different locations and/or sites. Thus, site-specific data may be considered for the determination of the CCI. In particular in that e.g., an academic hospital or a specialty hospital for e.g. cardiovascular diseases generally requires a more detailed analysis than a primary care center providing care for an outpatient population. The type of site (clinic) may be identified by its location or position. Therefore, the location may be considered for CCI determination.

Calibration may alternatively or in addition relate to adapting or calibrating texts, which have been generated by using different linguistic concepts. For this purpose, an ontology may be used to transfer different terms with the same or similar meaning into a common term. An example for an ontology is the Fleischer Society: Glossary of Terms for Thoracic Imaging https://pubs.rsna.org/doi/10.1148/radiol.232558. The term "Nodule" could serve as an example.

The calibration task may refer to a computer-implemented task for calibrating data of different patients for a similar medical question or a similar annotation task, data of the same patients, but from different studies and/or procedures and/or from different image acquisition times and/or data of reports from different authors (language transfer).

Alternatively or in addition calibration may relate to:

- Population with high vs. low percentage of normal cases
- Population with high vs low percentage of cases requiring comparison of current and prior studies
- Differences in image acquisition and reconstruction, e.g.

   ◦ low-dose vs standard dose chest CT (standard dose having better image quality),
   ◦ chest CT with 1mm slice thickness vs 3mm slice thickness (3mm has fewer images)

- Different procedure mix and/or

- Different reporting standards, e.g. reports where all findings (normal and abnormal) are described in detail vs reports which only mention abnormal findings.

Alternatively or in addition, the NN may comprise at least one of a vision language model, a large language model, and/or an image processing model.

In another aspect, the invention relates to a method for calculating a case complexity index, CCI, for an input dataset, in particular comprising at least one medical image and optionally a related report by using a trained neural network, NN, which has been trained with a method as described above. This aspect refers to the inference phase, during which the trained NN is applied for unseen cases.

The input dataset may be preprocessed. In particular, the input dataset may be calibrated by means of a calibration module or a calibration algorithm adapted for use in the inference phase, in particular for harmonizing the input dataset.

Input data comprise image data, which are acquired on a medical scanner (CT, MRI, US, PET etc.). The raw data may be subject to post processing to provide the respective images. The images are typically stored in a local or cloud-based memory. The images are subject to further processing by means of executing downstream tasks. Downstream tasks may be implemented by software and/or hardware. A typical downstream task may comprise an annotation task.

Before executing the downstream task on a computing device, the method for calculating the CCI may be used. In particular, the CCI may be used to configure the computing entity for the specific task with estimated resources, which are estimated on the basis of the CCI. The CCI may be used to control the computing entity and/or the data transfer to the same on the basis of the CCI (the CCI indicating bandwidth and data transmission requirements). The CCI may be used to control computing resources of the computing entity, which is selected to execute the downstream task. The CCI may alternatively or in addition be used to select a computing entity which has the appropriate resources according to the CCI (e.g., software licenses and/or processing power and/or other resources required for complex cases, etc.). The CCI may be used to configure a device and/or software, implemented on that device for a downstream task.

In particular:
The CCI could be used to trigger other devices to perform additional image reconstructions from raw imaging data, e.g. trigger image reconstruction of a chest CT with thinner slice thickness based on the case complexity.

The CCI may be used to select among a multitude of AI devices which AI device(s) should be used for a given study, e.g. a case with high complexity due to extensive infectious disease in the lungs may not benefit from an algorithm intended to detect pulmonary nodules.

The CCI may be used to select which AI operating point to use for a given study, e.g. high-sensitivity or high-specificity mode. For example, an imaging study with low complexity due to lungs without presence of disease patterns could trigger to run an algorithm for detection of pulmonary nodules at a higher sensitivity operating point.

The CCI could also be used to sort the reading worklist in which a clinician should read the images, e.g. starting with the most complex cases at the beginning of the reading session.

Cases with very high complexity could be added to additional reading worklists for reading by two clinicians.

The CCI may be used to select cases which could be used as input for AI algorithm training and store them in a designated location for this purpose which can be shared with the AI developer after de-identification.

The CCI may be used to select cases for quality assurance processes and store them in a designated location for this purpose.

In addition or alternatively, the determination of the CCI may consider a typical long tail distribution of findings, see also Kahn et al. Thus, it is be taken into account that there is a small set of common findings and a large set of uncommon or rare findings. The CCI could be tailored (configured) to assign higher complexity to rare findings compared to common finding, in particular with respect to the long tail distribution.

The preprocessing may in addition or alternatively comprise an uncertainty module, which is configured for calibrating uncertainties in the report. For example, slightly different formulations in the report may still refer to the same content or same formulations in the report may refer to different imaging findings. Also, reports are expected have inherent inter-reader variability due to perception and interpretation challenges. This is taken into account when training and/or applying the NN in the inference phase.

The determined CCI may be used for configuring a downstream task, to be executed on the medical image(s), in particular an annotation task.

The downstream task may require certain resources. In particular, the downstream task (e.g., annotation) may require certain computing resources. Depending on the CCI, different computing resources (hardware-based and/or software-based) may be allocated for the downstream task, e.g., with respect to:

- Resolution of an image visualization device, e.g., monitor,
- processing capacities (e.g., for image processing),
- allocation of time for the task,
- human resources with a certain skill level,
- software licenses (needed for the downstream task)
- bandwidth for data transfer (both uplink and/or downlink) and/or
- further resources, in particular hardware resources.

The downstream task may be configured or controlled by evaluating the CCI. In particular, the computing resources, mentioned above, like processing capacities and/or the resolution of the e.g. monitor may be configured or the appropriate computing resources are selected based on the CCI. Thus, the appropriate computing resources may be determined and allocated as a preparatory measure to enhance the whole procedure. Alternatively or in addition, it is possible to configure the computing resources on a system, which has been identified to be available. Thus, e.g., variable computing resources may be adapted according to the CCI. For example, software licenses and bandwidth and/or other resources may be switched on or off or may be added or reduced according to actual need, which is determined according to the determined CCI.

Further, the required execution time for the downstream task, in particular the annotation time, may be estimated and/or the skill level of the e.g. annotation task may be estimated. Thus, the present invention serves to improve quality by allocating the appropriate resources (time, skill level of the annotator, and/or computing resources for annotation) for the downstream tasks, in particular annotation task.

Generally, a downstream task, may comprise or be an image-related medical processing task, like a reporting task or a task required for reporting, like an annotation task, an image processing task, a selection task and/or another software-based task, which is to be executed on the image.

In another aspect, the invention relates to a computing device for executing the training method as mentioned above.

In another aspect, the invention relates to a CCI-calculator device, which is configured to calculate a case complexity index, CCI. The CCI-calculator device may be configured to execute a method for calculating the case complexity index as described above. The CCI-calculator device may comprise: an input interface for receiving an input dataset, an output interface which is configured to provide the calculated case complexity index, CCI and a memory for storing a trained neural network, which has been trained with a method as described above with any alternative implementation.

In another aspect, the invention relates to a computer system for use in medical technology for applying a case complexity index, CCI, for an image-related medical processing task, comprising:

- An input interface for receiving an input dataset, in particular at least one medical image and optionally a related report;
- A calculator interface to a CCI-calculator device as described above for calculating the case complexity index, CCI;
- A control interface for controlling the image-related medical processing task based on the calculated CCI.

The CCI-calculator device may be part of the system. For example, the CCI-calculator device may be part of or may be implemented on the computing device (entity) which is configured to calculate the CCI. In addition or alternatively, the CCI-calculator device may be part of the system but implemented on another computing entity which is (only) connected via data connection (wired or wireless, like via an internet-based protocol, or LAN, WLAN and/or radio transmission). In this case, the system comprises a calculator interface for connecting to the CCI calculator device. It is also possible, that the CCI calculator device is connected to the system with an internal connection.

The CCI-calculator device may be part of the computing entity on which the downstream task is to be executed. Alternatively, the CCI-calculator device may be implemented on the image acquisition device or a related computing device.

The system may be implemented in a distributed manner, so that part thereof are implemented on a first computing entity and other parts thereof may be implemented on another computing entity. For example, the trained NN of the CCI-calculator device may be implemented locally or remotely.

In another aspect, the invention relates to a computer program product comprising program elements which cause a computing device to perform the steps of the method for training a NN or the steps of the method for calculating the CCI as described above when the program elements are loaded into a memory of the computing device.

In another aspect, the invention relates to a computer-readable medium having stored thereon program elements which can be read and executed by a computing device to perform steps of the method for training a NN or the steps of the method for calculating the CCI as described above when the program elements are executed by the computing device.

Brief description of the figures

Fig. 1 is a schematic flow chart of a training method according to an embodiment of the present invention;

Fig. 2 is a schematic flow chart of a method for calculating the case complexity index, CCI according to an embodiment of the present invention;

Fig. 3 is a block diagram, schematically representing a case complexity index calculator device according to an embodiment of the present invention;

Fig.4 shows an overview figure of a system for applying the CCI;

Fig. 5 shows a training phase and several example embodiments of a training method;

Fig. 6 shows an inference phase and several example embodiments of a method for calculating the CCI;

Fig. 7 shows a possible implementation of a system for using the determined CCI and

Fig. 8 shows by way of example a table in matrix strucutre for calcualted CCIs for different input data.

Description of the drawings

The present invention refers to methods and means for providing an objective metric for calculating a complexity index for a medical image and to detemine such a complexity index, in the form of a case complexity index, CCI.

Medical images usually require a set of subsequent tasks to be performed after imaging, which may also be denoted as downstream tasks, like annotating, selecting in particular for report generation, evaluating and others? Other potential downstream tasks may include:

- Image reconstruction tasks (e.g. in CT: different kernels, slice thickness, projections),
- Image analysis for all compartments in the images including e.g. detection, characterization, measurement of findings,
- Comparison of current and prior studies,
- Report generation,
- Archiving of imaging data and results and/or
- Quality assurance.

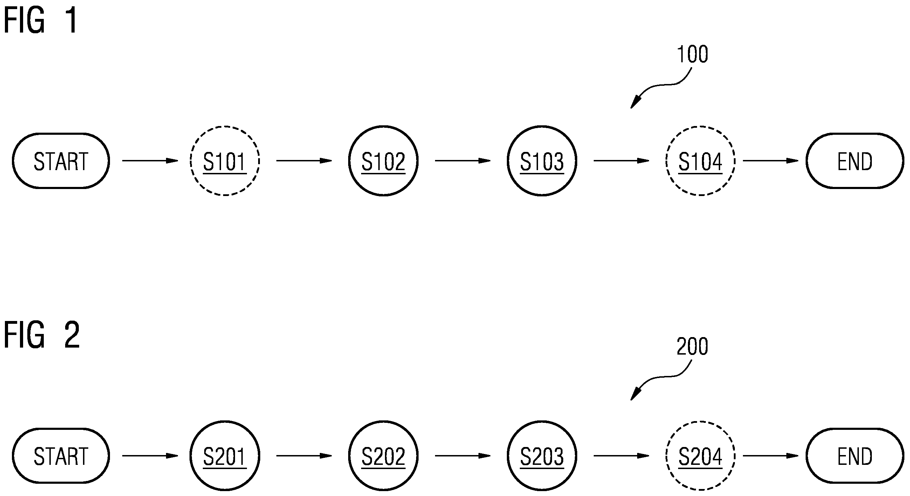

The present invention refers inter alia to a computer-implemented method 100 for training a neural network for determining a case complexity index, CCI, which is represented in Fig. 1.

After Start of the method, in an optional step (shown in dotted lines) S101 the training data may be preprocessed before beeing used for training, in particular the training data may be calibrated.

In step S102 training data (in raw or preprocessed form) are received. Training data may comprise of a medical image I out of a set of medical images (like a study for a patient). In an embodiment, the training data may in addition comprise a report, which refers or relates to said image. The training data may further comprise a CCI for the medical image. The CCI used in training may be determined algorithmically or manually and is associated to the image I.

In step S103 the NN is trained for providing a trained NN, which is configured for determining the CCI for a medical image I and optionally for a related report R by adjusting weights and biases of the NN such that a loss function is minimized.

In an optional step S104 the trained neural network is provided and may be used in inference for new (unseen) images I to determine the CCI for the image I.

After this, the method may end or may be trained on new data.

Fig. 2 shows a method 200 for using the trainded model, in particular the trained NN, in an inference phase. After start of the method, in step S201 an input dataset is received. The input dataset comprises at least one medical image I and may optionally comprise a medical report for said image. In other embodiments, the NN may be trained on additional data, like clinical data, operational data and/or guideline data. Thus, these data types may serve as input dataset as well in inference phase. In step S202 the trained NN is used for calculating the CCI for the received image I. In step S203 the calculated CCI is provided as output. This may be done on a human machine userinterface, HMI and/or on other output devices. In addition or alternatively, the calculated CCI may be transferred to other computing entities for further processing. The calculated CCI may optionally be used in step S204 (shown in dotted lines, because not mandatory) to configure and/or control downstream processes, in particuarl software-based or software-supported processes on the image I, like an annotation task. After this, the method may be reiterated or may end. Retraining is also possible.

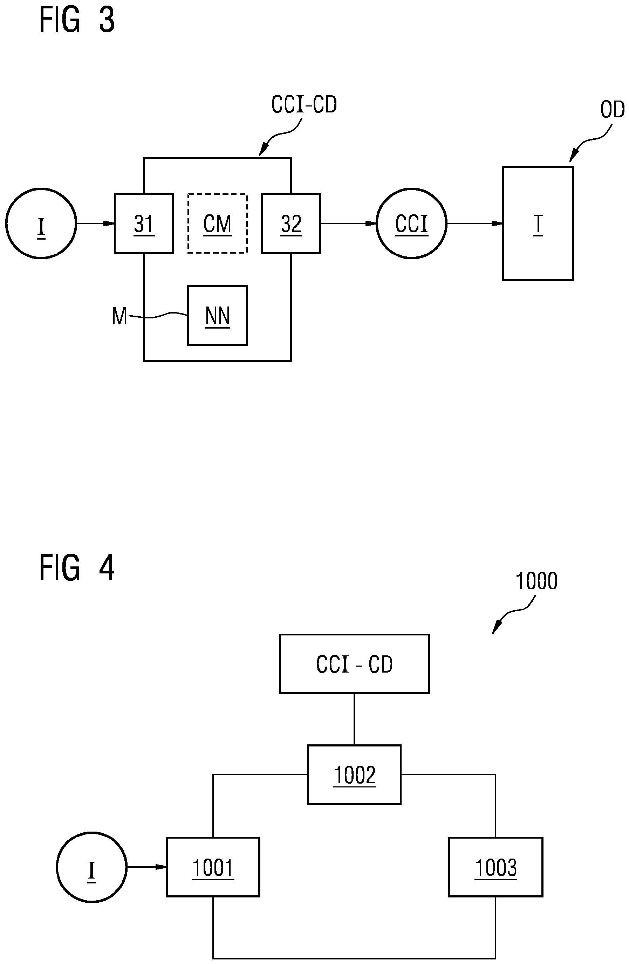

Fig. 3 shows a schematic drawing of a CCI-calculator device CCI-CD, which is configured to calculate a case complexity index, CCI. The CCI-calculator device CCI-CD may be configured to execute the method 200 for calculating a CCI. The CCI-calculator device CCI-CD may be implemented as hardware and/or software. The CCI-calculator device CCI-CD may be implemented on a CPU or GPU. The CCI-calculator device CCI-CD may comprise an input interface 31 for receiving an input dataset; an output interface 32 which is configured to provide the calculated case complexity index, CCI; and a memory M for storing a trained neural network NN, which has been trained with a training method as described above. The CCI-calculator device CCI-CD may be interconnected to other devices OD, which are configured to execute the downstream task T, like an annotation task. The calculated case complexity index, CCI may be used to open or closed loop control or configure the downstream task T. The CCI-calculator device CCI-CD may comprise a calibration module CM on an optional basis (dotted lines).

Fig. 4 shows an example computer system 1000 system for use in medical technology for applying a case complexity index, CCI, for an image-related medical processing task, comprising an input interface 1001 for receiving an input dataset, in particular comprising at least one medical image I and optionally a related report R; a calculator interface 1002 to a CCI-calculator device CCI-CD as mentioned above for calculating the case complexity index, CCI; and a control interface 1003 for controlling the image-related medical processing task (downstream task T) based on the calculated CCI.

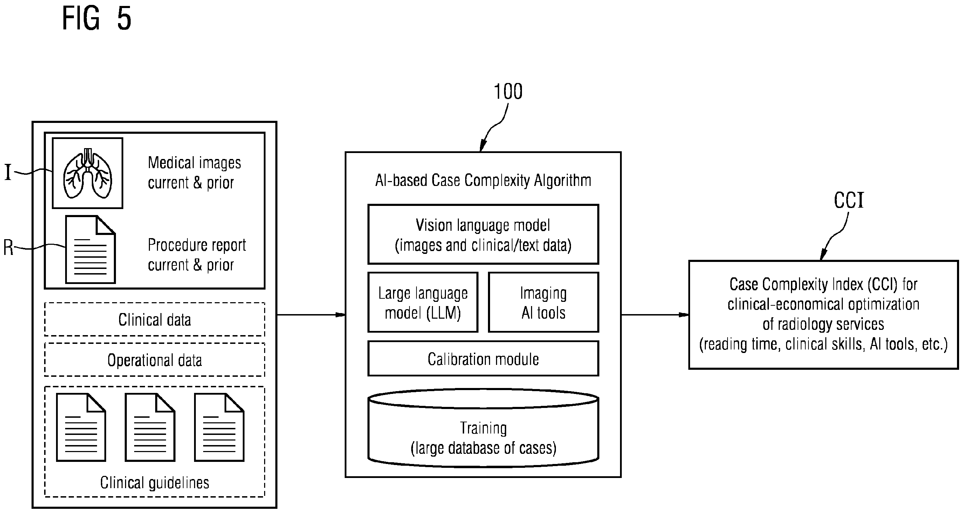

Fig. 5 shows an example embodiment of the training method 100. Input to the training in form of training data are at least images. Alternatively training data may further comprise a report R for the image. Thus, a pair of training data may be provided, namely pairs of images and (e.g. procedure) reports. Alternatively or optionally further training data may be provided and processed, in particular clinical data, operational data and/or clinical guidelines (optional data). Clinical data can include e.g. patient demographics, known diagnoses, co-morbidities, therapy related information and lab values. Operational data in the retrospective training phase can include experience/skills of reading physicians or medical annotators, reading/annotating times as well as referral sources and external data, such as Relative Value Units, RVU, for an exam.

Relative Value Units (RVU) are assigned to specific procedures to determine physician reimbursement and do reflect factors such as time, intensity and cost of care. RVUs can also be used to determine physician productivity. However, RVU are determined by a committee approach with an update cycle of several years. Comprehensive and continuous analysis of all relevant input data with the CCI may enable more granular and accurate results to drive clinical-economical optimization, e.g. assignment of cases to available readers.

Joint training on imaging and text data can be done with vision-language processing approaches. For more details it is referred to https://www.frontiersin.org/articles/10.3389/frai.2019.00028/full.

The presence of complementary and contradicting information between image data and reports can be utilized to determine case complexity across large numbers of cases even without knowing the exact ground truth in each case. Procedure reports may also contain inaccuracies and errors.

The reporting style (structure, level of detail, terminology) is expected to vary across physicians and sites. Different words may have the same meaning while small differences in wording can imply very different clinical meaning. Use of "uncertainty language" in imaging procedure reports is very common, e.g. "lung nodule can't be ruled out". Therefore, reporting style and uncertainty language in retrospective procedure reports can be processed by large language models (LLM) and used in the training of the CCI algorithm, i.e. the training method.

Comparison with prior images is standard of care and can be trained using retrospective cases with current and prior exams. This includes the impact of priors on reading time when compared to cases without prior exams.

Training may include approaches to distinguish cases with common findings from cases with rare findings in the long-tail distribution without the explicit need to detect or characterize the specific finding or disease.

Imaging AI tools may include algorithms to detect cases with specific findings as well as cases with non-actionable disease.

The Calibration Module CM can be implemented as solving an optimization problem. The Calibration Module CM can be configured taking into account site specific data.

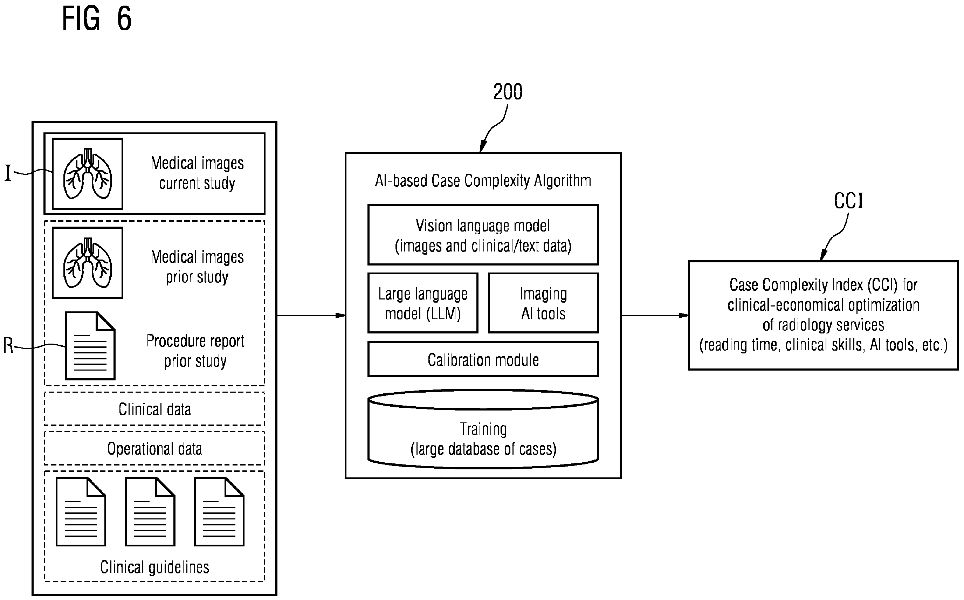

Fig. 6 shows the method 200 for calculating a CCI in an inferene or deployment phase. The method 200 or algorithm processes medical images from the current study which need to be read (required input) as well as images and reports from the prior study or studies, and optionally clinical data, operational data and/or clinical guidelines (optional data). The input dataset comprise medical images I and optionally a related medical report R. Thus, the input dataset may comprise of a multimodal dataset, consiting of image data and text data. In this scenario, the training method 100 needs to be trained with images I and reports R as well.

If the input dataset only comprises images I in the inference method 200, the training method 100 correspondingly needs to be trained with images.

Prior studies can refer to the same or a related procedure type. For example, a current chest radiograph may have a chest CT as the prior exam.

Clinical data in the prospective deployment phase can include the reason for the exam, known diagnoses, and related information such as lab values.

Operational data in the prospective deployment phase can include e.g. the referral source for the procedure.

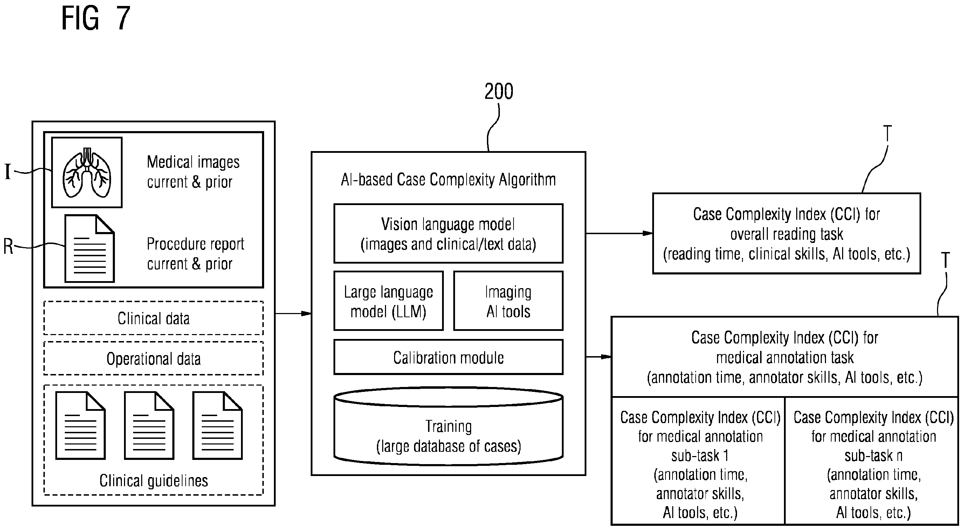

Fig. 7 shows in an example embodiment of the invention, the use of the calculated CCI for the downstream tasks T performed by medical annotators performing image review and/or annotation services towards a radiology report or medical image.

The calculated CCI may be used to match the skills of the annotators (e.g. basic non-physician, advanced non-physician, radiologist, subspecialty radiologist) to specific cases or tasks and to measure productivity while adjusting for case difficulty.

The CCI for the overall annotation task T (e.g. annotating a chest CT with all findings) could be broken down into multiple annotation sub-tasks (e.g. detecting lung nodules, measuring their size, etc.).

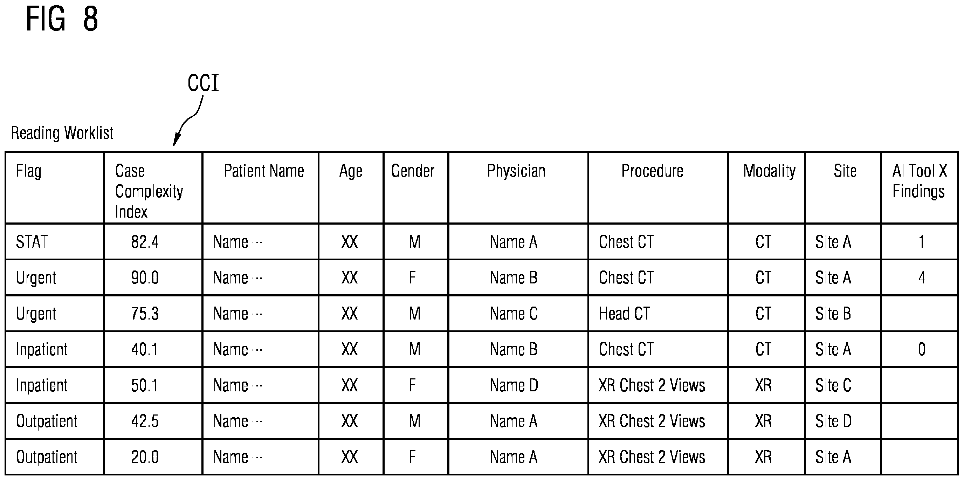

Fig. 8 showns an exemplary representation of a calculated case complexity index, CCI. The CCI may be part of the reading worklist of an image service provider. The CCI can be used across different imaging studies and multiple sites. The CCI can also be used by an imaging service provider serving multiple customers sending cases for reading (e.g. teleradiology service), where customer requirements for reading and reporting may vary substantially. This also includes RaaS offerings using medical annotators to prepare preliminary reports.

In one embodiment of the invention, the CCI can be used for specific imaging procedures, e.g. chest CT exams or chest radiographs.

In one embodiment of the invention, the CCI provides a sub-index for reading time and reading difficulty.

The AI-based calculation of the case complexity index, CCI can be systematically and proactively used for technical optimization, such as optimized assignment of cases to readers and/or configuration of devices for the execution of the downstream task T and/or worklist prioritization. The proposed CCI has the potential to be highly accurate due to the comprehensive scope of input data. The proposed CCI is also sustainable over time as the training can be updated to reflect technology improvements, new reporting requirements or other learnings from providing imaging services.

The CCI can be used as part of a Radiology as a Service implementation to execute reading tasks as efficiently as possible while ensuring highest possible quality of care. It can also be provided to customers as part of an imaging IT solution.

**Claims**

1. Computer-implemented method (100) for training a neural network, NN, for determining a case complexity index, CCI, comprising the following method steps:

   - receiving (S102) training data, comprising:

     - a medical image (I) of a set of medical images and optionally a related report (R) for the medical image;
     - a CCI for the medical image (I);

   - training (S103) the NN for providing a trained NN, which is configured for determining the CCI for a medical image (I) by adjusting weights and biases of the NN such that a loss function is minimized.

2. Method (100) according to claim 1, wherein the training data further comprise at least one of the following data elements:

   - clinical data for the respective patient, the medical image refers to;
   - operational data and/or
   - guideline data.

3. Method (100) according to any one of the preceding claims, wherein the set of medical images comprises current images and/or prior images of the same or of a related procedure, in particular of the same patient.

4. Method (100) according to any one of the preceding claims, wherein the method comprises:

   - Using a calibration module (CM), which is configured for calibrating (S101) the received training data, in particular with respect to different images and/or reports.

5. Method (100) according to any one of the preceding claims, wherein the NN comprises at least one of a vision language model, a large language model, and/or an image processing model.

6. Method (200) for calculating a case complexity index, CCI, for an input dataset, in particular comprising at least one medical image (I) and optionally a related report (R) for the medical image (I) by using (S202) a trained neural network, NN, which has been trained with a method according to any of the preceding method (100) claims.

7. Method (200) according to the directly preceding claim, wherein the calculated CCI is used for configuring (S204) a software-based downstream task on the medical image (I), in particular an image annotation task, in particular with respect to estimated reading time, and/or clinician skill level.

8. CCI-calculator device (CCI-CD), which is configured to calculate a case complexity index, CCI, comprising:

   - An input interface (31) for receiving an input dataset;
   - An output interface (32) which is configured to provide a calculated case complexity index, CCI;
   - A memory for storing a trained neural network, which has been trained with a method according to any of claims 1 - 6.

9. Computer system (1000) for use in medical technology for applying a case complexity index, CCI, for an image-related medical processing task, comprising:

   - An input interface (1001) for receiving an input dataset, in particular comprising at least one medical image (I) and optionally a related report (R);
   - A calculator interface (1002) to a CCI-calculator device (CCI-CD) according to claim 8 for calculating the case complexity index, CCI;
   - A control interface (1003) for controlling the image-related medical processing task based on the calculated CCI.

10. Computer program product comprising program elements which cause a computing device to perform the steps of the method (100) for training a neural network, NN according to any of the preceding method claims 1 - 5 or of the method (200) for calculating a case complexity index, CCI, according to claim 6 or 7 when the respective program elements are executed by the computing device.

11. A computer-readable medium having stored thereon program elements which can be read and executed by a computing device to perform the steps of the method (100) for training a neural network, NN according to any of the preceding method claims 1 - 5 or of the method (200) for calculating a case complexity index, CCI, according to claim 6 or 7 when the respective program elements are loaded into a memory of the computing device.

**Amended claims in accordance with Rule 137(2) EPC.**

1. Computer-implemented method (100) for training a neural network, NN, for determining a case complexity index, CCI, comprising the following method steps:

   - receiving (S102) training data, comprising:

      - a medical image (I) of a set of medical images and optionally a related report (R) for the medical image;
      - a CCI for the medical image (I);

      - training (S103) the NN for providing a trained NN, which is configured for determining the CCI for a medical image (I) by adjusting weights and biases of the NN such that a loss function is minimized;

   wherein the CCI is calculated on the basis of time for reading the medical image (I).

2. Method (100) according to claim 1, wherein the training data further comprise at least one of the following data elements:

   - clinical data for the respective patient, the medical image refers to;
   - operational data and/or
   - guideline data.

3. Method (100) according to any one of the preceding claims, wherein the set of medical images comprises current images and/or prior images of the same or of a related procedure, in particular of the same patient.

4. Method (100) according to any one of the preceding claims, wherein the method comprises:

   - Using a calibration module (CM), which is configured for calibrating (S101) the received training data, in particular with respect to different images and/or reports.

5. Method (100) according to any one of the preceding claims, wherein the NN comprises at least one of a vision language model, a large language model, and/or an image processing model.

6. Method (200) for calculating a case complexity index, CCI, for an input dataset, in particular comprising at least one medical image (I) and optionally a related report (R) for the medical image (I) by using (S202) a trained neural network, NN, which has been trained with a method according to any of the preceding method (100) claims, and wherein the CCI is calculated on the basis of time for reading the at least one medical image (I).

7. Method (200) according to the directly preceding claim, wherein the calculated CCI is used for configuring (S204) a software-based downstream task on the medical image (I), in particular an image annotation task, in particular with respect to estimated reading time, and/or clinician skill level.

8. CCI-calculator device (CCI-CD), which is configured to calculate a case complexity index, CCI, comprising:

   - An input interface (31) for receiving an input dataset;
   - An output interface (32) which is configured to provide a calculated case complexity index, CCI, wherein the CCI is calculated on the basis of time for reading a medical image (I);
   - A memory for storing a trained neural network, which has been trained with a method according to any of claims 1 - 6.

9. Computer system (1000) for use in medical technology for applying a case complexity index, CCI, for an image-related medical processing task, comprising:

   - An input interface (1001) for receiving an input dataset, in particular comprising at least one medical image (I) and optionally a related report (R);
   - A calculator interface (1002) to a CCI-calculator device (CCI-CD) according to claim 8 for calculating the case complexity index, CCI;
   - A control interface (1003) for controlling the image-related medical processing task based on the calculated CCI.

10. Computer program product comprising program elements which cause a computing device to perform the steps of the method (100) for training a neural network, NN according to any of the preceding method claims 1 - 5 or of the method (200) for calculating a case complexity index, CCI, according to claim 6 or 7 when the respective program elements are executed by the computing device.

11. A computer-readable medium having stored thereon program elements which can be read and executed by a computing device to perform the steps of the method (100) for training a neural network, NN according to any of the preceding method claims 1 - 5 or of the method (200) for calculating a case complexity index, CCI, according to claim 6 or 7 when the respective program elements are loaded into a memory of the computing device.

EP 4 672 260 A1

## FIG 1

100

$$\text{START} \rightarrow S101 \rightarrow S102 \rightarrow S103 \rightarrow S104 \rightarrow \text{END}$$

## FIG 2

200

$$\text{START} \rightarrow S201 \rightarrow S202 \rightarrow S203 \rightarrow S204 \rightarrow \text{END}$$

## FIG 3

CCI-CD

OD

I → 31 [ CM ] 32 → CCI → T

M — NN

## FIG 4

1000

CCI - CD

1002

I → 1001

1003

## FIG 5

Medical images current & prior

Procedure report current & prior

Clinical data

Operational data

Clinical guidelines

100

AI-based Case Complexity Algorithm

Vision language model (images and clinical/text data)

Large language model (LLM)

Imaging AI tools

Calibration module

Training (large database of cases)

CCI

Case Complexity Index (CCI) for clinical-economical optimization of radiology services (reading time, clinical skills, AI tools, etc.)

EP 4 672 260 A1

# FIG 6

**200**

I — Medical images current study

Medical images prior study

R — Procedure report prior study

Clinical data

Operational data

Clinical guidelines

AI-based Case Complexity Algorithm

Vision language model (images and clinical/text data)

Large language model (LLM)

Imaging AI tools

Calibration module

Training (large database of cases)

**CCI**

Case Complexity Index (CCI) for clinical-economical optimization of radiology services (reading time, clinical skills, AI tools, etc.)

## FIG 7

200

| Medical images current & prior | AI-based Case Complexity Algorithm | Case Complexity Index (CCI) for overall reading task (reading time, clinical skills, AI tools, etc.) |

I → Medical images current & prior

R → Procedure report current & prior

Clinical data

Operational data

Clinical guidelines

**AI-based Case Complexity Algorithm**

Vision language model (images and clinical/text data)

Large language model (LLM)

Imaging AI tools

Calibration module

Training (large database of cases)

T

Case Complexity Index (CCI) for overall reading task (reading time, clinical skills, AI tools, etc.)

T

Case Complexity Index (CCI) for medical annotation task (annotation time, annotator skills, AI tools, etc.)

| Case Complexity Index (CCI) for medical annotation sub-task 1 (annotation time, annotator skills, AI tools, etc.) | Case Complexity Index (CCI) for medical annotation sub-task n (annotation time, annotator skills, AI tools, etc.) |

EP 4 672 260 A1

## FIG 8

Reading Worklist

CCI

| Flag | Case Complexity Index | Patient Name | Age | Gender | Physician | Procedure | Modality | Site | AI Tool X Findings |
|------|------|------|------|------|------|------|------|------|------|
| STAT | 82.4 | Name ··· | XX | M | Name A | Chest CT | CT | Site A | 1 |
| Urgent | 90.0 | Name ··· | XX | F | Name B | Chest CT | CT | Site A | 4 |
| Urgent | 75.3 | Name ··· | XX | M | Name C | Head CT | CT | Site B | |
| Inpatient | 40.1 | Name ··· | XX | M | Name B | Chest CT | CT | Site A | 0 |
| Inpatient | 50.1 | Name ··· | XX | F | Name D | XR Chest 2 Views | XR | Site C | |
| Outpatient | 42.5 | Name ··· | XX | M | Name A | XR Chest 2 Views | XR | Site D | |
| Outpatient | 20.0 | Name ··· | XX | F | Name A | XR Chest 2 Views | XR | Site A | |

EP 4 672 260 A1

Europäisches
Patentamt
European
Patent Office
Office européen
des brevets

# EUROPEAN SEARCH REPORT

Application Number

EP 24 18 3908

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 11 282 196 B2 (IBM [US]) 22 March 2022 (2022-03-22) * col 2 par 3, col 6 par 3, par spanning col 6 and 7, col 8 par 3, col 18 last par, col 20 last par, col 21 par 2 * ----- | 1-11 | INV. G16H50/20 G16H50/30 G16H50/70 |

TECHNICAL FIELDS
SEARCHED     (IPC)

G16H

The present search report has been drawn up for all claims

| Place of search | Date of completion of the search | Examiner |
|---|---|---|
| Munich | 22 November 2024 | Bankwitz, Robert |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
...................................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 18 3908

This annex lists the patent family members relating to the patent documents cited in the above-mentioned European search report.
The members are as contained in the European Patent Office EDP file on
The European Patent Office is in no way liable for these particulars which are merely given for the purpose of information.

22-11-2024

| Patent document cited in search report | | Publication date | Patent family member(s) | | Publication date |
|---|---|---|---|---|---|
| US 11282196 | B2 | 22-03-2022 | US | 2020160510 A1 | 21-05-2020 |
| | | | US | 2020327668 A1 | 15-10-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KAHN CE**. The Long Tail. *Radiology Artificial Intelligence*, 18 April 2019, https://pubs.rsna.org/page/ai/blog/2019/4/the_long tail

- *Guidelines for Management of Incidental Pulmonary Nodules Detected on CT Images: From the Fleischner Society*, 2017, https://pubmed.ncbi.nlm.nih.gov/28240562/